# EUROPEAN PATENT APPLICATION

(11) **EP 4 524 245 A1**
(43) Date of publication of application: **19.03.2025**
(21) Application number: 22941146.7
(22) Date of filing: 12.05.2022
(51) Int. Cl.: C12N 11/089, C12N 9/10, C12N 9/20, C12N 9/06, C12N 9/04, C12N 9/02

(54) **IMMOBILIZED ENZYME, PREPARATION METHOD THEREFOR, AND USE THEREOF**

(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville North Carolina 27560 (US); ZHANG, Na, Tianjin 300457 (CN); RAJASEKAR, Vyasarayani Williams, Morrisville North Carolina 27560 (US); CUI, Yuxia, Tianjin 300457 (CN); ZHAO, Jiadong, Tianjin 300457 (CN); ZHANG, Chunyue, Tianjin 300457 (CN)
(74) Representative: Delbarba, Andrea
(86) International application number: PCT/CN2022/092506
(87) International publication number: WO 2023/216190

(57) **Abstract**

The present application provides an immobilized enzyme, a preparation method therefor and an application thereof. The immobilized enzyme includes an epoxy resin carrier and an enzyme, the enzyme and the epoxy resin carrier are linked by a covalent bond, and the epoxy resin carrier is an LX-109S epoxy resin. The LX-109S epoxy resin is used as the epoxy resin carrier in the present application, such that on the basis of characteristics of the carrier itself, an immobilization effect of the carrier on the enzyme is more stable, and covalent bonding with the enzyme is firm without affecting the activity of the enzyme itself.

## Description

### Technical Field

The present application relates to the technical field of immobilization of enzymes, and specifically relates to an immobilized enzyme, a preparation method therefor and an application thereof.

### Background

Biocatalysis has become an important part in green synthesis of drugs, which is also one of the most promising technologies for catalyzing structural units and intermediates of the drugs and especially provides a unique alternative method for chiral synthesis. More and more biological enzymes are used as catalysts in industrial processes, but due to mild use conditions of the enzymes and easy denaturation of the enzymes themselves, the enzymes have extremely harsh requirements on the environment and are difficult to recycle, such that industrial application of the enzymes is greatly limited, and accordingly, demands for immobilized enzymes are increased. Therefore, the immobilized enzymes, usually referred to as "biocatalysts", are widely used in industrial organic synthesis and biotransformation.

Preparation methods for the immobilized enzymes generally include adsorption, covalent coupling, crosslinking and embedding. The covalent coupling method includes covalently bonding inactive side chain groups of enzymes to active functional groups in carriers, such as carboxylic acid carriers, amino carriers, or epoxy carriers. The epoxy carriers have high reactivity and plasticity, and epoxy groups on the carriers can directly react with inactive groups, such as -NH₂, -HS, etc., on enzyme molecules for covalent immobilization. Because the epoxy carriers have extremely high reactivity, multi-point immobilization can be performed on enzymes containing a variety of inactive groups to improve the bonding strength between the enzymes and the carriers. Therefore, the epoxy carriers have extremely high superiority in the immobilization of enzymes.

In recent years, synthetic chemists have increasing interest in continuous flow synthesis, and currently, the continuous flow synthesis has also attracted attention in biotransformation. The emergence of novel immobilized platforms realizes seamless integration of the immobilized enzymes in continuous flow biocatalysis. The discovery and evolution of novel efficient enzymes, novel reverse synthesis methods focusing on biocatalysis, cost reduction of recombinant proteins and enzyme immobilization strategies have all made full preparation for the continuous flow biocatalysis.

Epoxy resins have been widely used in the immobilization of enzymes, but the resins have a good immobilization effect on purified pure enzymes and an unsatisfactory immobilization effect on crude enzyme solutions, such that costs of the immobilized enzymes are increased. Moreover, violent chemical reactions occurring in a covalent bonding process are usually extremely unfavorable to the activity of biomolecules, and thus will affect the enzyme activity in an immobilization process of enzymes with more active activity centers, leading to an extremely low activity recovery rate. In addition, covalent bonding of the epoxy carriers and the enzymes needs to be performed at high ionic strength. When the ionic strength is not suitable or many interfering factors are involved in the environment, the enzymes and the carriers are usually bonded only in the form of ionic adsorption, stable chemical bonding cannot be formed, and the reusability is poor.

### SUMMARY

The main purpose of the present application is to provide an immobilized enzyme, a preparation method therefor and an application thereof, so as to solve the problem of an unstable immobilization effect of epoxy resin immobilized enzymes in the prior art.

In order to achieve the above purpose, according to one aspect of the present application, an immobilized enzyme is provided. The immobilized enzyme includes an epoxy resin carrier and an enzyme, the enzyme and the epoxy resin carrier are linked by a covalent bond, the epoxy resin carrier is an LX-109S epoxy resin, and the enzyme is derived from a crude enzyme.

Further, the enzyme is selected from any one of a transaminase, a D-lactate dehydrogenase, a cyclohexanone monooxygenase, a ketoreductase, an ene reductase, a nitrilase, an ammonia lyase, an amino acid dehydrogenase, an imine reductase, a lipase, and mutants thereof, the ammonia lyase is preferably selected from any one of a phenylalanine ammonia lyase, and the amino acid dehydrogenase is preferably selected from any one of a leucine dehydrogenase or a phenylalanine dehydrogenase.

Further, in the immobilized enzyme, a loading amount of the enzyme on the epoxy resin carrier per gram is 30 mg to 70 mg.

Further, the immobilized enzyme further includes a cofactor.

According to another aspect of the present application, a preparation method for the immobilized enzyme according to any one of the above statements is provided. The preparation method includes: Step S1, mixing a first phosphate buffer solution with an enzyme to form an enzyme solution, wherein the enzyme is a crude enzyme; and Step S2, mixing the enzyme solution with an epoxy resin for an immobilization reaction and then performing washing with a second phosphate buffer solution to obtain the immobilized enzyme, wherein the epoxy resin is an LX-109S epoxy resin.

Further, a volume ratio of the enzyme solution to the epoxy resin is 3:1 to 5:1.

Further, a pH value of the first phosphate buffer solution is 7.0 to 8.0, the first phosphate buffer solution includes sodium chloride, and a concentration of the sodium chloride in the first phosphate buffer solution is preferably 1±0.2 mol/L; and a pH value of the second phosphate buffer solution is 7.0 to 8.0, and the second phosphate buffer solution does not include sodium chloride.

Further, the Step S2 includes: mixing the enzyme solution with the epoxy resin at 10-20°C, performing culturing on a shaker for 16-24 h, and then performing standing and incubation at 3-5°C for 40-48 h to obtain an immobilized system; and washing the immobilized system with the second phosphate buffer solution to obtain the immobilized enzyme.

According to another aspect of the present application, an application of the immobilized enzyme according to any one of the above statements is provided. The application includes applying the immobilized enzyme as a catalyst in a continuous catalytic reaction.

According to the technical schemes applied in the present application, the LX-109S epoxy resin is used as the epoxy resin carrier in the present application, such that on the basis of characteristics of the carrier itself, an immobilization effect of the carrier on the enzyme is more stable, and covalent bonding with the enzyme is firm without affecting the activity of the enzyme itself. Therefore, a crude enzyme can be directly used as an enzyme source, thereby greatly simplifying an enzyme immobilization process, reducing a production cost of the immobilized enzyme and shortening a process flow.

### Detailed Description of the Embodiments

It should be noted that embodiments in the present application and features in the embodiments can be combined with each other without conflict. The present application is described in detail below in conjunction with the embodiments.

As analyzed in the background of the present application, an immobilization effect of epoxy resin immobilized enzymes in the prior art is unstable. In order to solve the problem, the present application provides an immobilized enzyme, a preparation method therefor and an application thereof.

In a typical embodiment of the present application, an immobilized enzyme is provided. The immobilized enzyme includes an epoxy resin carrier and an enzyme, the enzyme and the epoxy resin carrier are linked by a covalent bond, and the epoxy resin carrier is an LX-109S epoxy resin.

The LX-109S epoxy resin is used as the epoxy resin carrier in the present application, such that on the basis of characteristics of the carrier itself, an immobilization effect of the carrier on the enzyme is more stable, and covalent bonding with the enzyme is firm without affecting the activity of the enzyme itself. Therefore, a crude enzyme can be directly used as an enzyme source, thereby greatly simplifying an enzyme immobilization process, reducing a production cost of the immobilized enzyme and shortening a process flow.

Based on covalent bonding between the epoxy resin and the enzyme, all enzymes that can achieve covalent bonding and immobilization in the prior art can be considered for application in the present application. Especially, when the enzyme is selected from any one of a transaminase, a D-lactate dehydrogenase, a cyclohexanone monooxygenase, a ketoreductase, an ene reductase, a nitrilase, an ammonia lyase, an amino acid dehydrogenase, an imine reductase, a lipase, and mutants thereof, the immobilization effect is particularly prominent. The ammonia lyase is preferably selected from any one of a phenylalanine ammonia lyase, and the amino acid dehydrogenase is preferably selected from any one of a leucine dehydrogenase or a phenylalanine dehydrogenase. Some enzyme sources are listed and shown in Table 1 below.

**Table 1**

| Abbreviation | Enzyme | Source | Amino acid sequence |
|---|---|---|---|
| TA IV-Ss (parent) | Transaminase | *Aminotransferase IV from Sciscionella sp. SE31* | *Wp_031466213.1* |
| | | | SEQ ID NO: 1: MTTTEFANSNLVAVEPGAIRE PTPPGSVIQYSEYELDRSQPLAGGVAWIEGEY VPADEARISIFDTGFGHSDLTYTVAHVWHGNIF RLEDHLDRLLHGAARLKLETGMSREELAGIAK RCVSLSQLREAYVNITITRGYGKKRGEKDLTKL THQVYVYAIPYLWAFPPEEQIFGTSVIVPRHVR RAGRNTIDPTIKNYQWGDLTAASFEAKDRGAR SAVLLDADNCVAEGPGFNVVLVKDGALVSPSR NALPGITRKTVYEIAAAKGIETMLRDVTSSELYE ADELMAVTTAGGVTPITSLDGEQVGNGEPGPI TVAIRDRFWALMDEPSSLIEAIDY |
| TA IV-Ss-1 (mutant) | Transaminase | | Mutation on the basis of SEQ ID NO. 1: G69Y+H70T+L73A+V77G+A78I+Y130V+K 141S+S142T+R143P+G144Y+L148A+L151 A+T1S2R+L163Q+A165I+S207I+F208R+K2 11H+T290S+A292G+12 amino acids increased in N-termal +F11I+G17V+Q40H+T204S+T66M+A151E+ V130M |

**Table 1-continued**

| Abbreviation | Enzyme | Source | Amino acid sequence |
|---|---|---|---|
| TA IV-Ss-2 (mutant) | Transaminase | | Mutation on the basis of SEQ ID NO: 1: G17V+Q40H+T66M+G69Y+H70T+L73A+V 77G+A781+Y130V+K141S+S142T+R143P+ G144Y+L148A+L151E+T152R+L163Q+A16 5I+T204S+S207I+F208R+K211H+T290S+A 292G+12 amino acids increased in N-termal +F-11I+E151H+H153P+E145G-K146R+Y19 8F+V130M+S204N+S278R+V244H |
| CR-Ac | Ketoreductase | Carbonyl reductase from *Acetobacter sp. CCTCC M209061* | *AWE05150.1* |
| | | | SEQ ID NO: 2: MTRVAGKVAIVSGAANGIG KATAQLLAKEGAKVVIGDLKEEDGQKAVAEI KAAGGEAAFVKLNVTDEAAWKAAIEQTLKLY GRLDIAVNNAGIAYSGSVESTSLEDWRRVQ SINLDGVFLGTQVAIEAMKKSGGGSIVNLSSI EGLIGDPMGAAYNASKGGVRLFTKSAALHC AKSGYKIRVNSVHPGYIWTPMVAGLTKEDA AARQKLVDLHPIGHLGEPNDIAYGILYLASDE SKFVTGSELVIDGGYTAQ |
| CHMO-Rr | Cyclohexanon e monooxygena se | Cyclohexanone monooxygenase from *Rhodococcus ruber-SD1* | *AAL14233. 1* |
| | | | SEQ ID NO: 3: MTTSIDREALRRKYAEERD KRIRPDGNDQYIRLDHVDGWSHDPYMPITP REPKLDHVTFAFIGGGFSGLVTAARLRESGV ESVRIIDKAGDFGGVWYWNRYPGAMCDTA AMVYMPLLEETGYMPTEKYAHGPEILEHCQ RIGKHYDLYDDALFHTEVTDLVWQEHDQRW RISTNRGDHFTAQFVGMGTGPLHVAQLPGI PGIESFRGKSFHTSRWDYDYTGGDALGAP MDKLADKRVAVIGTGATAVQCVPELAKYCR ELYVVQRTPSAVDERGNHPIDEKWFAQIATP GWQKRWLDSFTAIWDGVLTDPSELAIEHED LVQDGWTALGQRMRAAVGSVPIEQYSPEN VQRALEEADDEQMERIRARVDEIVTDPATAA QLKAWFRQMCKRPCFHDDYLPAFNRPNTH LVDTGGKGVERITENGVVVAGVEYEVDCIVY ASGFEFLGTGYTDRAGFDPTGRDGVKLSEH WAQGTRTLHGMHTYGFPNLFVLQLMQGAA LGSNIPHNFVEAARVVAAIVDHVLSTGTSSV ETTKEAEQAWVQLLLDHGRPLGNPECTPGY YNNEGKPAELKDRLNVGYPAGSAAFFRMM DHWLAAGSFDGLTFR |
| ERED-Chr | Ene Reductase | *Old yellow enzyme from Cinyseobacterum sp. CA49* | *ALE60336.1* |
| | | | SEQ ID NO: 4: MSTESLFTPFKYKNLELKN RIVMAPMTRAQSDNGVPTQQIADYYARRAA AEVGLILSEGTVINRPASKNMQNIPDFYGTE ALNGWKNVIDAVHHNGGKMGPQIWHVGDT RSTPDYPLEDMEKASTMTLEDIQDTIAQFAA SAKSAKDLGFDVLEIHGAHGYLIDQFFWEGT NTRTDEYGGKTIKERSRFAVDVVKAIRAAVG EDFTIIIRLSQWKQQDYSVKLAHTPEEMEEW LLPLKDAGVDIFHCSQRRFWEPEFEGSDLN FAGWAKKITGQPTITVGSVGLEGDFMAAFG GQGTEKADLTELTKRLERGDFDLVAVGRAL LQDPEWAKKVKEQNTEALLDFSAESLGVLY |
| IRED-1 | Imine Reductase | oxidoreductase from Amycolatopsis decaplanina | *WP_007034301.1* |
| | | | SEQ ID NO: 5: MSPGGTLTLGDLTVSRMG YGAMRLSGPGIWGPPSDRETAIAVLREAVE LGVTHIDTSDFYGPHTVNELIREALHPYPDEL HIVTKVGAKRSPDKGWPSALSREELTSAVH DNLRNLGVDVLDVVNLRLAGEHGVFPIPVSI TEPFEVLAELRQQGLIRHLGLSHVSAEQVKE ARAIAPVVCVQNEYNVANRANDDLLDALAAI NIPFVPYFPLGGFTPLQSGVLDDCARRVDAT PMQVALAWLLQRSPNILVIPGTSSPSHLREN VAAAKLELPADVIADLDALV |
| IRED-2 | Imine Reductase | Imine reductase from Bacilus cereus (WP_095755701.1) | *WP 095755701.1* |
| | | | SEQ ID NO: 6: MTDNALAQPGPSTPLTLLG TGAMGTALARAWLAAGHPVTVWNRTPARA EALAAEGATVAASAAEAVAANRLVVVCLLDD ASVGEALDGADLTGRDLVNITTGTPGQGRS RAAWAKARGARFLDGGIMAVPPMIGAPDSG AYVFYSGSAALFEEHRDTLAVPAGTTYVGA DPGFAALHDVALLSAMNGMFAGITHAFALIR REDIAPKDFAPLLVSWLTAMAHSAHKAADQ LESGDYGKDVVSSLAMQVAGNATLLRTAEE QGVSAELLRPYMDLMERRLALGNGEEDTTG VVELLLRKP |
| AADH-Ti | Leucine Dehydrogenas e | Thermostable Phenylalanine dehydrogenase (TIPDH) from *Thermoactinomyces imtermedius ATCC33205* | *P22823.1* |
| | | | SEQ ID NO: 7: MRDVFEMMDRYGHEQVIF CRHPQTGLKAIIALHNTTAGPALGGCRMIPY ASTDEALEDVLRLSKGMTYKCSLADVDFGG GKMVIIGDPKKDKSPELFRVIGRFVGGLNGR FYTGTDMGTNPEDFVHAARESKSFAGLPKS YGGKGDTSIPTALGVFHGMRATARFLWGTD QLKGRVVAIQGVGKVGERLLQLLVEVGAYC KIADIDSVRCEQLKEKYGDKVQLVDVNRIHK ESCDIFSPCAKGGVVNDDTIDEFRCLAIVGS ANNQLVEDRHGALLQKRSICYAPDYLVNAG GLIQVADELEGFHEERVLAKTEAIYDMVLDIF HRAKNENITTCEAADRIVMERLKKLTDIRRIL LEDPRNSARR SEQ ID NO: 8: MLPSGSDPAFSQPKSVLD AGLTCQGASPSSVSKPILLVPGTGTTGPQSF DSNWIPLSTQLGYTPCWISPPPFMLNDTQV NTEYMVNAITALYAGSGNNKLPVLTWSQGG LVAQWGLTFFPSIRSKVDRLMAFAPDYKGT VLAGPLDALAVSAPSVWQQTTGSALTTALR NAGGLTQIVPTTNLYSATDEIVQPQVSNSPL |
| CALB (parent) | Lipase | Candida antarctica (P41365.1) CALBd | SEQ ID NO: 8: MLPSGSDPAFSQPKSVLD AGLTCQGASPSSVSKPILLVPGTGTTGPQSF DSNWIPLSTQLGYTPCWISPPPFMLNDTQV NTEYMVNAITALYAGSGNNKLPVLTWSQGG LVAQWGLTFFPSIRSKVDRLMAFAPDYKGT VLAGPLDALAVSAPSVWQQTTGSALTTALR NAGGLTQIVPTTNLYSATDEIVQPQVSNSPL |
| | | | DSSYLFNGKNVQAQAVCGPLFVIDHAGSLT SQFSYVVGRSALRSTTGQARSADYGITDCN PLPANDLTPEQKVAAAALLAPAAAAIVAGPK QNCEPDLMPYARPFAVGKRTCSGIVTP |
| CALB-1 (mutant) | Lipase | | Mutation on the basis of SEQ ID NO: 8: V155L+I190L+L145G |
| CALB-2 (mutant) | Lipase | | Mutation on the basis of SEQ ID NO: 8: V155L+I190L+L145G+L141V+I286M |

Due to the characteristics of the LX-109S epoxy resin, the epoxy resin can not only be used for immobilization of purified enzymes, especially enzymes derived from crude enzymes (namely unpurified enzymes), but also still achieve a good immobilization effect. For example, the enzyme is derived from a crude enzyme solution expressed by *Escherichia coli* engineering bacteria, and the crude enzyme solution is a mixed protein solution obtained by precipitating cells, re-suspending the cells with a phosphate buffer solution, crushing the cells by a lysozyme, ultrasound or homogenization, and then removing crushed cell residues by centrifugation.

It should be explained that an enzyme production process usually includes introducing an exogenous gene into a host cell to construct engineered bacteria, performing recovery and expansion culture of strains, performing high-density fermentation, crushing cells, collecting an enzyme protein solution after removing cell debris, purifying a target enzyme protein by column purification, and performing freeze-drying on the purified enzyme solution to obtain a pure enzyme powder. However, the crude enzyme in the present application refers to a crude enzyme powder formed by directly freeze-drying a crude enzyme solution obtained before column purification without purification treatment. Usually, the crude enzyme includes endogenous proteins expressed in a host cell background, including a protease, an oxidase, a reductase, etc., and factors for translation of water-soluble endogenous proteins, amino acids, nucleic acids, etc. A proportion of the target enzyme in the crude enzyme solution is very small and is usually less than 30%, and a viscosity of the enzyme solution is large. The crude enzyme solution is directly used to immobilize the resin carrier of the enzyme protein, although the cost is low, due to the effect of miscellaneous proteins and other substances, the immobilization bonding rate is low, and the immobilized enzyme often shows relatively low activity.

As found through experimental research, the LX-109S epoxy resin for immobilizing the unpurified crude enzyme, compared with the immobilization effect of ECR8285 and LX120 epoxy resins on the crude enzyme, increases the enzyme activity and the reusability by 20% to 60%. An LX-109S epoxy resin immobilized lipase reacting in an aqueous phase and an organic phase, compared with immobilization of other types of resins, increases the enzyme activity and the reusability by 20% to 100%. And an LX-109S epoxy resin immobilized transaminase applied in enzymatic synthesis of sitapliptin, compared with the immobilization effect of other types of resins, increases the enzyme activity and the reusability by 20% to 60%.

On the basis of covalent bonding between the enzyme and the epoxy resin, full exertion of the catalytic activity of the enzyme is ensured on the basis of ensuring that a loading amount of the enzyme is increased as much as possible, and preferably, in the immobilized enzyme, the loading amount of the enzyme on the epoxy resin carrier each gram is 30 mg to 70 mg, for example, 30 mg, 35 mg, 40 mg, 45 mg, 50 mg, 55 mg, 60 mg, 65 mg, or 70 mg.

In some embodiments of the present application, in order to improve the catalytic activity and catalytic stability of the immobilized enzyme, preferably, the immobilized enzyme may further include an optional cofactor, and specific selection of the cofactor can be performed according to a specific type of the enzyme. For example, the cofactor is selected from PLP, FAD, NAD⁺, NADP⁺, etc. The specific selection is not listed one by one herein.

In another typical embodiment of the present application, a preparation method for the immobilized enzyme according to any one of the above statements is provided. The preparation method includes: Step S1, mixing a first phosphate buffer solution with an enzyme to form an enzyme solution, wherein the enzyme is a crude enzyme; and Step S2, mixing the enzyme solution with an epoxy resin for an immobilization reaction and then performing washing with a second phosphate buffer solution to obtain the immobilized enzyme, wherein the epoxy resin is an LX-109S epoxy resin.

In the present application, covalent bonding between the enzyme and the LX-109S epoxy resin is realized with the preparation method, and the preparation method is simple, easy to operate and easy to apply and popularize.

In some embodiments of the present application, in order to increase an immobilization rate of the enzyme in the enzyme solution, preferably, a volume ratio of the enzyme solution to the epoxy resin is 3:1 to 5:1, for example, 3:1 to 4:1, 3:1 to 3.5:1, 3.5:1 to 5:1, 3.5:1 to 4:1, or 4:1 to 5:1.

In some embodiments of the present application, the first phosphate buffer solution and the second phosphate buffer solution may be the same or different; preferably, the two are different in composition to play the role at each stage; preferably, a pH value of the first phosphate buffer solution is 7.0 to 8.0; and a pH value of the second phosphate buffer solution is 7.0 to 8.0. The basic composition of the first phosphate buffer solution and the second phosphate buffer solution is similar to that of a phosphate buffer solution commonly used in the prior art, the first phosphate buffer solution includes sodium chloride, and preferably, a concentration of the sodium chloride in the first phosphate buffer solution is 1±0.2 mol/L, that is, a phosphate is used as a buffer pair, and the sodium chloride is used to adjust the ionic strength. The second phosphate buffer solution does not include sodium chloride.

In order to improve the immobilization efficiency and improve the immobilization stability as much as possible, the Step S2 preferably includes: mixing the enzyme solution with the epoxy resin at 10-20°C, performing culturing on a shaker for 16-24 h, and then performing standing and incubation at 3-5°C for 40-48 h to obtain an immobilized system; and washing the immobilized system with the second phosphate buffer solution to obtain the immobilized enzyme. The culturing on a shaker may be performed with a common shaker apparatus, such as a rail shaker, in the prior art.

In some embodiments, the mixing is performed at a temperature of 10°C, 20°C, 15°C, 12°C, 18°C, 16°C, or 14°C; the culturing on a shaker is performed for 16 h, 17 h, 18 h, 19 h, 20 h, 21 h, 22 h, 23 h, or 24 h; and the standing is performed for 40 h, 41 h, 42 h, 43 h, 44 h, or 45 h.

In another typical embodiment of the present application, an application of the immobilized enzyme according to any one of the above statements is further provided. The application includes applying the immobilized enzyme as a catalyst in a continuous catalytic reaction. Because the immobilized enzyme of the present application has high stability, the catalytic activity in the continuous catalytic reaction is well maintained, and the service life is prolonged, thus ensuring the reaction efficiency of the continuous catalytic reaction.

Beneficial effects of the present application are further described below in combination with examples and comparative examples.

In the following examples, PB represents a phosphate buffer solution, and considering certain loss of an enzyme in an immobilization process, an amount of the enzyme used is higher than that of a free enzyme when an immobilized enzyme is prepared.

### Example 1: Immobilization process of a transaminase (TA IV-Ss) on an epoxy resin

1 g of an epoxy resin was taken out and washed with 0.2 M PB 8.0 and a 0.8 M NaCl buffer solution, the buffer solution was removed, and the resin was maintained for use. Then, 4 mL of an enzyme solution was added (the enzyme solution was prepared from a crude enzyme powder with 0.2 M PB 8.0 and a 0.8 M NaCl buffer solution to achieve a protein content of 25 mg/mL, with a corresponding cofactor PLP), cultured with the epoxy resin on a rail shaker at 20°C for 20 h, taken out, and subjected to standing and incubation at 4°C for 48 h. Washing was performed with 20 mM PB 8.0 for 3 times to obtain an immobilized transaminase loaded with 30-80 mg of a protein (a protein loading amount was measured by a Coomassie brilliant blue method using a microplate reader or a BCA (bicinchoninic acid) protein method, and due to different epoxy resins, different protein loading amounts were obtained).

Activity and reusability tests of an immobilized transaminase (TA IV-Ss-1 or TA IV-Ss-2) were as follows:

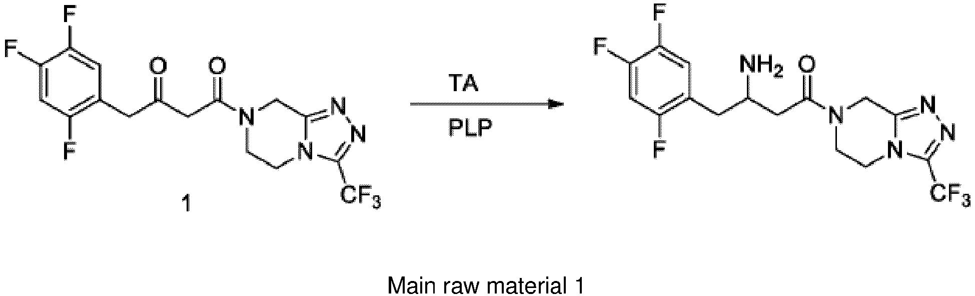

In a 20 mL reaction flask, 0.5 mL of MeOH was added to dissolve 0.1 g of a main raw material 1, 15 eq of isopropylamine hydrochloride and 15.0 mg of PLP (5'-pyridoxal phosphate) were added, 0.1 M PB 8.0 was supplemented until a final volume of a reaction solution was 5 mL, then 0.1 g of a TA IV-Ss-1 crude enzyme powder, 0.1 g of a TA IV-Ss-2 crude enzyme powder, or the immobilized transaminase prepared from 0.2 g of the TA IV-Ss-1 crude enzyme powder or 0.2 g of the TA IV-Ss-2 crude enzyme powder was added, and stirring was performed at 47°C for 20 h. A conversion rate of the system was detected by HPLC. After each round of reaction was completed, the immobilized enzyme was separated and put into a next round of reaction for reuse so as to investigate a reuse number. Reaction data of the transaminase are shown in Table 2 below.

**Table 2**

| Enzyme | Main raw material | Epoxy resin type | Enzyme loading mount mg/g | Conversion rate (%) | Cycle number |
|---|---|---|---|---|---|
| TAIV-Ss-1 | 1 | Free enzyme without a carrier | - | >97% | 1 |
| | | ECR8285 | 67 | >80% | 3 |
| | | LX-109S | 64 | >95% | 11 |
| | | ECR8204F | -- | 60%-70% | 2 |
| | | ECR8209F | -- | 60%-70% | 3 |
| | | LX-1000EP | -- | <20% | 1 |
| | | LX103B | -- | <50% | 2 |
| | | EP200 | -- | <40% | 2 |
| | | LX107S | 43 | 70%-80% | 3 |
| | | LX120 | 56 | >80% | 4 |
| | | LX1000SW | -- | <50% | 2 |
| | | LX1000SD | -- | <50% | 2 |
| | | ES-1 | -- | 60%-70% | 2 |
| | | ES103 | -- | 60%-70% | 2 |
| | | ES105 | -- | <40% | 1 |
| | | ES108 | -- | <40% | 1 |
| | | ES-101 | -- | <40% | 1 |
| | | HFA403M | 13 | <40% | 2 |
| | | EC-HFAM | 9 | <40% | 2 |
| | | LX1000HFA | -- | >80% | 1 |
| | | LX-SW-7 | -- | 60%-70% | 3 |
| | | HFA001 | -- | 60%-70% | 4 |

**Table 2-continued**

| Enzyme | Main raw material | Epoxy resin type | Enzyme loading mount mg/g | Conversion rate (%) | Cycle number |
|---|---|---|---|---|---|
| | | Free enzyme without a carrier | -- | >99% | 1 |
| | | ECR8285 | 53 | >98% | 5 |
| | | LX-109S | 58 | >95% | 15 |
| | | ECR8204F | -- | >75% | 4 |
| | | ECR8209F | -- | >75% | 3 |
| | | LX-1000EP | -- | 40%-60% | 2 |
| | | LX103B | -- | <50% | 2 |
| | | EP200 | -- | 30%-50% | 1 |
| TAIV-Ss-2 | 1 | LX107S | 45 | >80% | 5 |
| | | LX120 | 39 | >90% | 6 |
| | | LX1000SW | -- | 60%-80% | 4 |
| | | LX1000SD | -- | 50%-70% | 3 |
| | | ES-1 | -- | <60% | 2 |
| | | ES103 | -- | 30%-40% | 1 |
| | | ES105 | -- | <20% | 1 |
| | | ES108 | -- | <45% | 1 |
| | | ES-101 | -- | 30%-50% | 2 |
| | | HFA403M | -- | <20% | 1 |
| | | EC-HFAM | 11 | <20% | 1 |
| | | LX1000HFA | -- | 70%-80% | 4 |
| | | LX-SW-7 | -- | 30%-40% | 1 |
| | | HFA001 | -- | <50% | 2 |

### Example 2: Immobilization process of a lipase (CALB) on an epoxy resin

An immobilization method was the same as that in Example 1.

Activity and reusability tests of an immobilized lipase (CALB) were as follows:

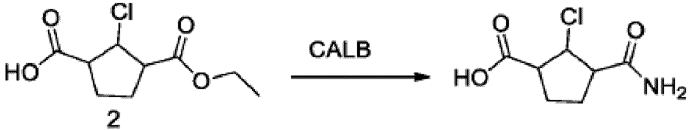

In a 10 mL reaction flask, 1.8 mL of MTBE was added to dissolve 0.1 g of a main raw material 2, 0.8 eq of HMDS (hexamethyldisilazane) was added, then 0.1 g of a CALB crude enzyme powder or an immobilized enzyme prepared from 0.1 g of the CALB crude enzyme powder was added, and stirring was performed at 40°C for 20 h. A conversion rate of the system was detected by HPLC. After each round of reaction was completed, the immobilized enzyme was separated and put into a next round of reaction for reuse so as to investigate a reuse number, which was recorded in Table 3.

Activity and reusability tests of an immobilized lipase (CALB-1 or CALB-2) were as follows:

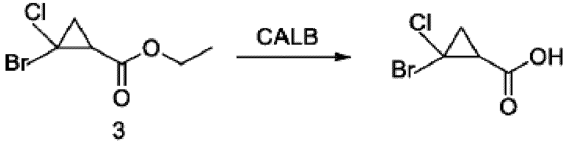

In a 10 mL reaction flask, 2 mL of MTBE was added to dissolve 0.1 g of a main raw material 3, 0.1 M KPB 7.5 was supplemented until a final volume of a reaction solution was 4 mL, then 0.1 g of a CALB-1 crude enzyme powder, a CALB-2 crude enzyme powder, or an immobilized enzyme prepared from 0.1 g of the CALB-1 crude enzyme powder or 0.1 g of the CALB-2 crude enzyme powder was added, and stirring was performed at 47°C for 16-20 h. A conversion rate of the system was detected by HPLC. After each round of reaction was completed, the immobilized enzyme was separated and put into a next round of reaction for reuse so as to investigate a reuse number. The raw material 3 was recorded in Table 3.

Activity and reusability tests of the immobilized lipase (CALB-1 or CALB-2) were as follows:

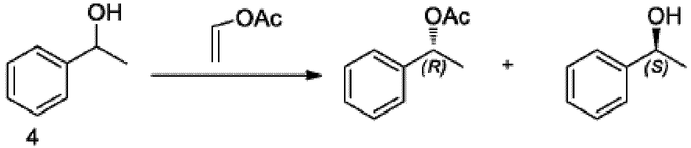

In a 10 mL reaction flask, 2 mL of MTBE was added to dissolve 0.1 g of a main raw material 4, 0.275 g of vinyl acetate was added, then 0.1 g of the CALB-1 crude enzyme powder, the CALB-2 crude enzyme powder, or the immobilized enzyme prepared from 0.1 g of the CALB-1 crude enzyme powder or 0.1 g of the CALB-2 crude enzyme powder was added, and stirring was performed at 30°C for 16-20 h. A conversion rate of the system was detected by HPLC. After each round of reaction was completed, the immobilized enzyme was separated and put into a next round of reaction for reuse so as to investigate a reuse number, which was recorded in Table 3.

**Table 3**

| Enzyme | Main raw material | Epoxy resin | Enzyme loading mount mg/g | Conversion rate (%) | Cycle number |
|---|---|---|---|---|---|
| CALB | | Free enzyme without a carrier | -- | <1% | 1 |
| | | ECR8285 | 39 | <40% | 2 |
| | | LX-1000EPN | 22 | <30% | 2 |
| | | LX-EPHA | -- | <30% | 1 |
| | | LX-109S | 43 | >99% | 25 |
| | | ECR8204F | -- | <20% | 2 |
| | | ECR8209F | -- | <30% | 2 |
| | | LX-1000EP | -- | <20% | 1 |
| | | LX103B | -- | <10% | 1 |
| | 2 | EP200 | -- | <5% | 1 |
| | | LX107S | 31 | <20% | 1 |
| | | LX120 | 35 | 60%-80% | 4 |
| | | LX1000SW | -- | <5% | 1 |
| | | LX1000SD | -- | <5% | 1 |
| | | ES-1 | -- | <5% | 1 |
| | | ES103 | 8 | <5% | 1 |
| | | ES105 | -- | <10% | 1 |
| | | ES108 | -- | <10% | 1 |
| | | ES-101 | -- | <10% | 1 |
| | | HFA403M | -- | <5% | 1 |
| | | EC-HFAM | -- | <5% | 1 |
| | | LX1000HFA | -- | <20% | 2 |
| | | LX-SW-7 | -- | <20% | 2 |
| | | HFA001 | -- | <20% | 2 |
| | | Amino resin | 34 | <20% | 1 |

**Table 3-continued 1**

| Enzyme | Main raw material | Epoxy resin | Enzyme loading mount mg/g | Conversion rate (%) | Cycle number |
|---|---|---|---|---|---|
| | | Free enzyme without a carrier | -- | >99% | 1 |
| | | ECR8285 | 43 | >85% | 5 |
| | | LX-1000EPN | 40 | >85% | 3 |
| | | LX-EPHA | 37 | >85% | 3 |
| | | LX-109S | 49 | >99% | 9 |
| | | ECR8204F | -- | <50% | 3 |
| | | ECR8209F | -- | <30% | 2 |
| | | LX-1000EP | -- | <40% | 4 |
| | | LX103B | -- | <50% | 2 |
| | | EP200 | -- | <5% | 2 |
| | | LX107S | 33 | 70-80% | 3 |
| CALB-1 | 3 | LX120 | 37 | >85% | 7 |
| | | LX1000SW | -- | <50% | 1 |
| | | LX1000SD | -- | <50% | 1 |
| | | ES-1 | -- | <5% | 1 |
| | | ES103 | 14 | <20% | 1 |
| | | ES105 | -- | <30% | 1 |
| | | ES108 | -- | <50% | 2 |
| | | ES-101 | -- | <30% | 1 |
| | | HFA403M | -- | <10% | 1 |
| | | EC-HFAM | -- | <10% | 1 |
| | | LX1000HFA | -- | <70% | 2 |
| | | LX-SW-7 | -- | <60% | 2 |
| | | HFA001 | -- | <20% | 4 |
| | | Amino resin | 41 | 75-90% | 4 |
| CALB-2 | 3 | Free enzyme without a carrier | -- | 99% | 1 |
| | | ECR8285 | 61 | >90% | 7 |
| | | LX-1000EPN | 61 | >85% | 5 |
| | | LX-EPHA | 52 | >85% | 5 |
| | | LX-109S | 66 | >95% | 14 |
| | | ECR8204F | -- | >75% | 4 |
| | | ECR8209F | -- | 50-70% | 3 |
| | | LX-1000EP | -- | 60-75% | 4 |
| | | LX103B | -- | 60-75% | 5 |
| | | EP200 | -- | <50% | 3 |
| | | LX107S | 41 | >85% | 6 |
| | | LX120 | 49 | >85% | 7 |
| | | LX1000SW | -- | 50-70% | 5 |
| | | LX1000SD | -- | <45% | 1 |
| | | ES-1 | -- | <50% | 2 |
| | | ES103 | 20 | 20-40% | 1 |
| | | ES105 | -- | >75% | 5 |
| | | ES108 | -- | 60-75% | 4 |
| | | ES-101 | -- | 40-60% | 3 |
| | | HFA403M | -- | <30% | 1 |
| | | EC-HFAM | -- | <20% | 1 |
| | | LX1000HFA | -- | 70-85% | 5 |
| | | LX-SW-7 | -- | 55-70% | 3 |
| | | HFA001 | -- | <20% | 1 |
| | | Amino resin | 54 | >80% | 5 |

**Table 3-continued 3**

| Enzyme | Main raw material | Epoxy resin | Enzyme loading mount mg/g | Conversion rate (%) | Cycle number |
|---|---|---|---|---|---|
| CALB-1 | 4 | Free enzyme without a carrier | -- | 95% | 1 |
| | | ECR8285 | 47 | <80% | 3 |
| | | LX-1000EPN | 39 | <70% | 2 |
| | | LX-EPHA | 37 | <80% | 3 |
| | | LX-109S | 54 | >95% | 5 |
| | | ECR8204F | -- | <60% | 3 |
| | | ECR8209F | -- | 50-65% | 2 |
| | | LX-1000EP | -- | <50% | 2 |
| | | LX103B | -- | <60% | 3 |
| | | EP200 | -- | 40-60% | 2 |
| | | LX107S | 39 | 65-80% | 3 |
| | | LX120 | 51 | >75% | 4 |
| | | LX1000SW | -- | 35-40% | 1 |
| | | LX1000SD | -- | 40-55% | 2 |
| | | ES-1 | -- | <30% | 1 |
| | | ES103 | -- | <40% | 1 |
| | | ES105 | -- | <30% | 1 |
| | | ES108 | 24 | 60-80% | 4 |
| | | ES-101 | -- | 45-75% | 3 |
| | | HFA403M | -- | <30% | 1 |
| | | EC-HFAM | -- | <20% | 1 |
| | | LX1000HFA | -- | <50% | 2 |
| | | LX-SW-7 | -- | 55-75% | 3 |
| | | HFA001 | -- | <20% | 1 |
| | | Amino resin | 47 | 50-80% | 3 |

**Table 3-continued 4**

| Enzyme | Main raw material | Epoxy resin | Enzyme loading mount mg/g | Conversion rate (%) | Cycle number |
|---|---|---|---|---|---|
| | | Free enzyme without a carrier | -- | 99% | 1 |
| | | ECR8285 | 53 | 70-85% | 3 |
| | | LX-1000EPN | 55 | <75% | 2 |
| | | LX-EPHA | 48 | 60-70% | 3 |
| CALB-1 | 4 | LX-109S | 54 | >95% | 6 |
| | | ECR8204F | -- | 60-70% | 3 |
| | | ECR8209F | -- | 60-70% | 2 |
| | | LX-1000EP | -- | <75% | 2 |
| | | LX103B | -- | <40% | 1 |
| | | EP200 | -- | <30% | 1 |
| | | LX107S | 42 | 60-75% | 3 |
| | | LX120 | 50 | 70-85% | 5 |
| | | LX1000SW | -- | <60% | 2 |
| | | LX1000SD | -- | 40-55% | 2 |
| | | ES-1 | -- | <30% | 1 |
| | | ES103 | 28 | <40% | 1 |
| | | ES105 | -- | <30% | 1 |
| | | ES108 | -- | <80% | 3 |
| | | ES-101 | -- | <60% | 2 |
| | | HFA403M | -- | <30% | 1 |
| | | EC-HFAM | -- | <20% | 1 |
| | | LX1000HFA | -- | 60-75% | 3 |
| | | LX-SW-7 | -- | <60% | 2 |
| | | HFA001 | -- | <20% | 1 |
| | | Amino resin | 50 | 75-95% | 4 |

### Example 3: Conversion rate and reusability tests of an immobilized ketoreductase (CR-Ac)

An immobilization method was the same as that in Example 1.

Activity and reusability tests of an immobilized enzyme (CR-Ac) were as follows:

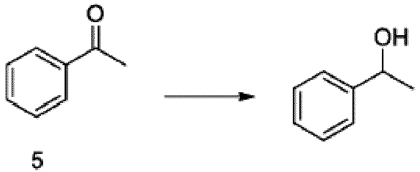

In a 10 mL reaction flask, 0.2 mL of isopropanol (IPA) was added to dissolve 0.1 g of a main raw material 5, 1 mL of 0.1 M PB 7.0 and 10 mg of NAD⁺ were added, then 0.1 g of a CR-Ac crude enzyme powder or an immobilized enzyme prepared from 0.2 g of the CR-Ac crude enzyme powder was added, and stirring was performed at 30°C for 20 h. A conversion rate of the system was detected by HPLC. After each round of reaction was completed, the immobilized enzyme was separated and put into a next round of reaction for reuse so as to investigate a reuse number. Reaction data are shown in Table 4.

**Table 4**

| Enzyme | Main raw material | Epoxy resin | Enzyme loading mount mg/g | Conversion rate (%) | Cycle number |
|---|---|---|---|---|---|
| | | Free enzyme without a carrier | -- | >99% | 1 |
| | | ECR8285 | 55 | >90% | 5 |
| | | LX-1000EPN | 51 | >80% | 2 |
| | | LX-EPHA | 38 | >80% | 3 |
| | | LX-109S | 53 | >95% | 9 |
| | | ECR8204F | -- | >80% | 3 |
| | | ECR8209F | -- | 70%-80% | 3 |
| | | LX-1000EP | -- | 60%-70% | 2 |
| | | LX103B | -- | <50% | 2 |
| | | EP200 | -- | <50% | 2 |
| | | LX107S | 42 | <20% | 1 |
| CR-Ac | 5 | LX120 | 49 | 70%-90% | 4 |
| | | LX1000SW | -- | <20% | 1 |
| | | LX1000SD | -- | <20% | 1 |
| | | ES-1 | -- | <30% | 1 |
| | | ES103 | -- | <10% | 1 |
| | | ES105 | -- | <10% | 1 |
| | | ES108 | -- | <10% | 1 |
| | | ES-101 | -- | <10% | 1 |
| | | HFA403M | -- | <40% | 2 |
| | | EC-HFAM | -- | <30% | 1 |
| | | LX1000HFA | 31 | <50% | 2 |
| | | LX-SW-7 | -- | <20% | 1 |
| | | HFA001 | -- | <50% | 2 |

### Example 4: Conversion rate and reusability tests of an immobilized monooxygenase (CHMO-Rr)

An immobilization method was the same as that in Example 1.

The activity of a CHMO-Rr epoxy carrier immobilized enzyme was detected by a reaction of a main raw material 6 below:

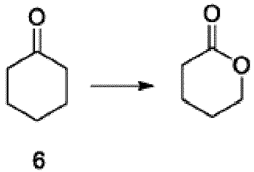

0.3 mL of isopropanol was loaded into a 10 mL reaction flask, 50 mg of the main raw material 6 and 3 mL of 0.1 M PB containing 5 mg of NADP⁺ (pH 8.0) were added, then 5 mg of an alcohol dehydrogenase (ABY93890.1) dry powder was added as a coenzyme, and 0.1 g of a CHMO-Rr crude enzyme powder or an immobilized enzyme prepared from 0.2 g of the CHMO-Rr crude enzyme powder was added. A reaction was carried out at 30°C for 20 h, and a conversion rate was tested. After each round of reaction was completed, the immobilized enzyme was separated and put into a next round of reaction for reuse so as to investigate a reuse number. Results are shown in the following Table 5.

**Table 5**

| Enzyme | Main raw material | Epoxy resin | Enzyme loading mount mg/g | Conversion rate (%) | Cycle number |
|---|---|---|---|---|---|
| | | Free enzyme without a carrier | -- | >99% | 1 |
| | | ECR8285 | 41 | 60%-70% | 2 |
| | | LX-1000EPN | 47 | <20% | 1 |
| | | LX-EPHA | 39 | <20% | 1 |
| | | LX-109S | 46 | >90% | 5 |
| | | ECR8204F | -- | <20% | 2 |
| | | ECR8209F | -- | <20% | 2 |
| | | LX-1000EP | -- | <20% | 2 |
| | | LX103B | -- | <10% | 1 |
| | | EP200 | -- | <10% | 1 |
| | | LX107S | 42 | <20% | 1 |
| CHMO-Rr | 6 | LX120 | 48 | 70%-90% | 3 |
| | | LX1000SW | -- | <20% | 1 |
| | | LX1000SD | -- | <20% | 1 |
| | | ES-1 | -- | <5% | 1 |
| | | ES103 | -- | <5% | 1 |
| | | ES105 | -- | <5% | 1 |
| | | ES108 | -- | <5% | 1 |
| | | ES-101 | -- | <5% | 1 |
| | | HFA403M | -- | <10% | 2 |
| | | EC-HFAM | -- | <20% | 1 |
| | | LX1000HFA | 19 | <30% | 2 |
| | | LX-SW-7 | -- | <5% | 1 |
| | | HFA001 | -- | <20% | 2 |

### Example 5: Conversion rate and reusability tests of an immobilized ene reductase (ERED-Sc)

An immobilization method was the same as that in Example 1.

The activity of an ERED-Sc epoxy carrier immobilized enzyme was detected by a reaction of a main raw material 7 below:

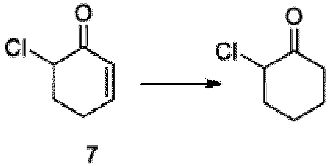

3 mL of 0.1 M PB (pH 7.0-8.0) was loaded into a 10 mL reaction flask, 0.1 g of the main raw material 7 was added, then 10 mg of NAD(P)⁺, 80 mg of ammonium formate, 20 mg of a glucose dehydrogenase (ACR78513.1) as a coenzyme were added, and 0.1 g of an EREC-Sc crude enzyme powder or an immobilized enzyme prepared from 0.2 g of the EREC-Sc crude enzyme powder was added. A reaction was carried out at 30°C for 20 h, and a conversion rate was tested. After each round of reaction was completed, the immobilized enzyme was separated and put into a next round of reaction for reuse so as to investigate a reuse number. Test results are shown in the following Table 6.

**Table 6**

| Enzyme | Main raw material | Carrier | Enzyme loading mount mg/g | Conversion rate (%) | Cycle number |
|---|---|---|---|---|---|
| | | Free enzyme without a carrier | -- | >99% | 1 |
| | | ECR8285 | 36 | >90% | 6 |
| | | LX-1000EPN | 33 | >90% | 4 |
| | | LX-EPHA | 38 | >90% | 5 |
| | | LX-109S | 42 | >98% | 11 |
| ERED-Sc | 7 | ECR8204F | -- | 70%-80% | 5 |
| | | ECR8209F | -- | 60%-70% | 6 |
| | | LX-1000EP | -- | 60%-70% | 4 |
| | | LX103B | -- | <50% | 3 |
| | | EP200 | -- | 50% | 2 |
| | | LX107S | 33 | 75% | 4 |
| | | LX120 | 37 | >85% | 5 |
| | | LX1000SW | -- | <40% | 2 |
| | | LX1000SD | -- | <40% | 2 |
| | | ES-1 | -- | <30% | 2 |
| | | ES103 | -- | <30% | 2 |
| | | ES105 | -- | <30% | 2 |
| | | ES108 | -- | <20% | 1 |
| | | ES-101 | -- | <20% | 1 |
| | | HFA403M | -- | <40% | 2 |
| | | EC-HFAM | -- | 50%-60% | 4 |
| | | LX1000HFA | 17 | 60%-70% | 5 |
| | | LX-SW-7 | -- | <20% | 1 |
| | | HFA001 | -- | <50% | 2 |

### Example 6: Conversion rate and reusability tests of an immobilized imine reductase (IRED)

An immobilization method was the same as that in Example 1.

The activity of an IRED-1 or IRED-2 epoxy carrier immobilized enzyme was detected by a reaction of a main raw material 8 below:

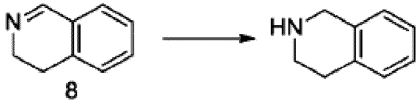

2 mL of a 0.1 M PB buffer solution (pH 7.0-8.0) was added to a 10 mL reactor, and then 100 mg of the main raw material 8, 10 mg of NAD⁺, 60 mg of ammonium formate, and 5 mg of an ammonium formate dehydrogenase (AIY34662.1) dry powder as a coenzyme were added. Then, 0.1 g of an IRED-1 crude enzyme powder, 0.1 g of an IRED-2 crude enzyme powder, or an immobilized enzyme prepared from 0.2 g of the IRED-1 crude enzyme powder or 0.2 g of the IRED-2 crude enzyme powder was added. A reaction was carried out at 30°C for 20 h, and a conversion rate was detected. After each round of reaction was completed, the immobilized enzyme was separated and put into a next round of reaction for reuse so as to investigate a reuse number. Results are shown in the following Table 7.

**Table 7**

| Enzyme | Main raw material | Carrier | Enzyme loading mount mg/g | Conversion rate (%) | Cycle number |
|---|---|---|---|---|---|
| | | Free enzyme without a carrier | -- | >99% | 1 |
| | | ECR8285 | 32 | 70%-90% | 5 |
| | | LX-1000EPN | 22 | 60%-70% | 2 |
| | | LX-EPHA | 17 | 60%-70% | 3 |
| | | LX-109S | 38 | >95% | 9 |
| | | ECR8204F | -- | 60%-70% | 5 |
| | | ECR8209F | -- | 70% | 6 |
| | | LX-1000EP | -- | 60%-70% | 3 |
| | | LX103B | -- | <50% | 2 |
| | | EP200 | -- | <50% | 2 |
| | | LX107S | 32 | 70%-80% | 4 |
| IRED-1 | 8 | LX120 | 34 | >85% | 4 |
| | | LX1000SW | -- | 75% | 3 |
| | | LX1000SD | -- | <20% | 1 |
| | | ES-1 | -- | <30% | 2 |
| | | ES103 | -- | <40% | 3 |
| | | ES105 | -- | <20% | 1 |
| | | ES108 | -- | <20% | 1 |
| | | ES-101 | 13 | <30% | 2 |
| | | HFA403M | 11 | <40% | 2 |
| | | EC-HFAM | -- | <40% | 1 |
| | | LX1000HFA | -- | >70% | 2 |
| | | LX-SW-7 | -- | 60%-70% | 4 |
| | | HFA001 | -- | <50% | 2 |

**Table 7-continued**

| Enzyme | Main raw material | Carrier | Enzyme loading mount mg/g | Conversion rate (%) | Cycle number |
|---|---|---|---|---|---|
| IRED-2 | 8 | Free enzyme without a carrier | -- | >99% | 1 |
| | | ECR8285 | 39 | >85% | 4 |
| | | LX-1000EPN | 29 | >75% | 3 |
| | | LX-EPHA | 25 | >75% | 4 |
| | | LX-109S | 33 | >90% | 8 |
| | | ECR8204F | -- | >75% | 4 |
| | | ECR8209F | -- | >70% | 4 |
| | | LX-1000EP | -- | >70% | 4 |
| | | LX103B | -- | 50-65% | 3 |
| | | EP200 | -- | 40-60% | 2 |
| | | LX107S | 27 | >70% | 4 |
| | | LX120 | 31 | 60-85% | 5 |
| | | LX1000SW | -- | 50-70% | 3 |
| | | LX1000SD | -- | 30-50% | 2 |
| | | ES-1 | -- | <20% | 1 |
| | | ES103 | -- | <30% | 1 |
| | | ES105 | -- | <20% | 1 |
| | | ES108 | -- | <30% | 1 |
| | | ES-101 | 8 | <40% | 2 |
| | | HFA403M | 15 | <20% | 1 |
| | | EC-HFAM | -- | <20% | 1 |
| | | LX1000HFA | -- | 50-70% | 3 |
| | | LX-SW-7 | -- | <50% | 2 |
| | | HFA001 | -- | <30% | 1 |

### Example 7: Conversion rate and reusability tests of an immobilized amino acid dehydrogenase (AADH-Ti)

An immobilization method was the same as that in Example 1.

The activity of an AADH-Ti epoxy carrier immobilized enzyme was detected by a reaction of a main raw material 9 below:

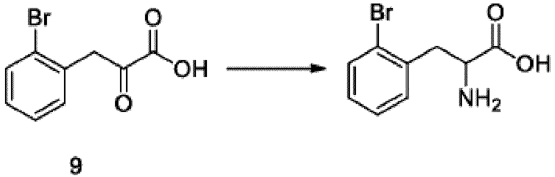

In a 10 mL reaction flask, 5 mL of 0.1 M Tris-Cl (pH 8.0-9.0) was added, 0.1 g of the main raw material 9 was added, 108 mg of ammonium chloride was added to adjust the pH value to 7.5-8.0, then 10 mg of NAD⁺ and 50 mg of GDH as a coenzyme were added, and 0.1 g of an AADH-Ti crude enzyme powder or an immobilized enzyme prepared from 0.2 g of the AADH-Ti crude enzyme powder was added. After a reaction was carried out at 30°C for 20 h, a conversion rate was tested. Test results are shown in the following Table.

**Table 8**

| Enzyme | Main raw material | Carrier | Enzyme loading mount mg/g | Conversion rate (%) | Cycle number |
|---|---|---|---|---|---|
| | | Free enzyme without a carrier | -- | >98% | 1 |
| | | ECR8285 | 44 | 70%-85% | 5 |
| | | LX-1000EPN | 45 | >50% | 2 |
| | | LX-EPHA | 38 | >80% | 4 |
| | | LX-109S | 49 | >90% | 8 |
| | | ECR8204F | -- | <40% | 3 |
| | | ECR8209F | -- | 30% | 3 |
| | | LX-1000EP | -- | 50% | 4 |
| | | LX103B | -- | <20% | 1 |
| | | EP200 | -- | 40% | 3 |
| | | LX107S | 39 | 60% | 4 |
| AADH-Ti | 9 | LX120 | 41 | >80% | 3 |
| | | LX1000SW | -- | <30% | 2 |
| | | LX1000SD | -- | <30% | 2 |
| | | ES-1 | -- | <20% | 1 |
| | | ES103 | -- | <10% | 1 |
| | | ES105 | -- | <10% | 1 |
| | | ES108 | -- | <20% | 1 |
| | | ES-101 | 9 | <30% | 1 |
| | | HFA403M | 13 | <30% | 2 |
| | | EC-HFAM | -- | <40% | 3 |
| | | LX1000HFA | -- | 60% | 4 |
| | | LX-SW-7 | -- | <20% | 1 |
| | | HFA001 | -- | 50% | 3 |

### Example 8 Application of a transaminase epoxy carrier immobilized enzyme in a continuous reaction of a packed bed

The transaminase TA IV-Ss-2 in Example 1 was immobilized to a carrier LX-109S, and an obtained immobilized enzyme was filled into a column reactor with a column volume of 120 mL, wherein a use amount of the immobilized enzyme was 70 g.

100 g of a main raw material 1 was dissolved in 0.5 L of methanol, 15 eq of isopropylamine hydrochloride (0.6 L of a 6 M isopropylamine hydrochloride aqueous solution) and 5 g of PLP were added, and a PB buffer solution (0.1 M, pH 8.0) was added to a constant volume of 5 L.

A flow rate was set at 0.4 mL/min, that is, a retention time was 300 min. A continuous reaction was carried out, and a conversion rate of effluent at an outlet end was detected. The conversion rate is greater than 98%, and after continuous operation for 280 h, the conversion rate is reduced to 86%. Specific details are shown in the following Table 9.

**Table 9**

| Enzyme | Carrier | Use amount of an immobilized enzyme | Column volume | Retention time | Operation time | Conversion rate |
|---|---|---|---|---|---|---|
| TA IV-Ss-2 | LX-109S | 70g | 120 mL | 300min | 280 h | 86% |

### Example 9 Application of a transaminase epoxy carrier immobilized enzyme in a reaction of a continuous stirring tank

The same immobilized enzyme TA IV-Ss-2 in Example 1 was used with LX-109S as a carrier. 60 g of the immobilized enzyme of a transaminase TA IV-Ss was added to a 1 L reactor, and 300 mL of a phosphate buffer solution was added.

4 L of PB (0.1 M, pH 8.0), 0.6 L of an isopropylamine hydrochloride aqueous solution (6 M) and 5 g of PLP were added to 100 g of a main raw material 1 for beating to prepare a suspension.

The substrate suspension was continuously added to a reaction flask at a rate of 0.4 mL/min (namely, a retention time of 500 min), and meanwhile, the reaction system was extracted at an outlet at the same flow rate (a filter head was added at a terminal end of a tube to prevent extraction of the immobilized enzyme). Under such conditions, a conversion rate can reach 90% or above, and after continuous operation for 350 h, the conversion rate is basically not reduced. Results are shown in the following Table 10.

**Table 10**

| Enzyme | Carrier | Use amount of an immobilized enzyme | Reactor volume | Retention time | Operation time | Conversion rate |
|---|---|---|---|---|---|---|
| TA IV-Ss-2 | LX-109S | 60g | 1 L | 500min | 350 h | >82% |

### Example 10: Immobilization process of a transaminase (TA IV-Ss) on an epoxy resin

1 g of an epoxy resin was taken out and washed with 0.2 M PB 8.0 and a 0.8 M NaCl buffer solution, the buffer solution was removed, and the resin was maintained for use. Then, 4 mL of a crude enzyme solution was added (the enzyme solution was prepared by dissolving a crude enzyme powder in 0.2 M PB 8.0 and a 0.8 M NaCl buffer solution to achieve a protein content of 20 mg/mL, with a corresponding cofactor PLP), cultured with the epoxy resin on a rail shaker at 20°C for 20 h, taken out, and subjected to standing and incubation at 4°C for 48 h. Washing was performed with 20 mM PB 8.0 for 3 times to obtain an immobilized transaminase containing 44 mg/g of a protein (a protein loading amount was measured by a Coomassie brilliant blue method using a microplate reader or a BCA (bicinchoninic acid) method).

### Example 11: Immobilization process of a transaminase (TA IV-Ss) on an epoxy resin

1 g of an epoxy resin was taken out and washed with 0.2 M PB 8.0 and a 0.8 M NaCl buffer solution, the buffer solution was removed, and the resin was maintained for use. Then, 4 mL of a crude enzyme solution was added (the enzyme solution was prepared by dissolving a crude enzyme powder in 0.2 M PB 8.0 and a 0.8 M NaCl buffer solution to achieve a protein content of 30 mg/mL, with a corresponding cofactor PLP), cultured with the epoxy resin on a rail shaker at 20°C for 20 h, taken out, and subjected to standing and incubation at 4°C for 48 h. Washing was performed with 20 mM PB 8.0 for 3 times to obtain an immobilized transaminase containing 51 mg/g of a protein (a protein loading amount was measured by a Coomassie brilliant blue method using a microplate reader or a BCA (bicinchoninic acid) method).

### Example 12: Immobilization process of a transaminase (TA IV-Ss) on an epoxy resin

1 g of an epoxy resin was taken out and washed with 0.2 M PB 8.0 and a 0.8 M NaCl buffer solution, the buffer solution was removed, and the resin was maintained for use. Then, 4 mL of a crude enzyme solution was added (the enzyme solution was prepared by dissolving a crude enzyme powder in 0.2 M PB 8.0 and a 0.8 M NaCl buffer solution to achieve a protein content of 25 mg/mL, with a corresponding cofactor PLP), cultured with the epoxy resin on a rail shaker at 10°C for 24 h, taken out, and subjected to standing and incubation at 4°C for 48 h. Washing was performed with 20 mM PB 8.0 for 3 times to obtain an immobilized transaminase containing 55 mg/g of a protein (a protein loading amount was measured by a Coomassie brilliant blue method using a microplate reader or a BCA (bicinchoninic acid) method).

### Example 13: Immobilization process of a transaminase (TA IV-Ss) on an epoxy resin

1 g of an epoxy resin was taken out and washed with 0.2 M PB 8.0 and a 0.8 M NaCl buffer solution, the buffer solution was removed, and the resin was maintained for use. Then, 4 mL of a crude enzyme solution was added (the enzyme solution was prepared by dissolving a crude enzyme powder in 0.2 M PB 8.0 and a 0.8 M NaCl buffer solution to achieve a protein content of 25 mg/mL, with a corresponding cofactor PLP), cultured with the epoxy resin on a rail shaker at 20°C for 16 h, taken out, and subjected to standing and incubation at 4°C for 48 h. Washing was performed with 20 mM PB 8.0 for 3 times to obtain an immobilized transaminase containing 48 mg/g of a protein (a protein loading amount was measured by a Coomassie brilliant blue method using a microplate reader or a BCA (bicinchoninic acid) method).

### Example 14: Immobilization process of a transaminase (TA IV-Ss) on an epoxy resin

1 g of an epoxy resin was taken out and washed with 0.2 M PB 8.0 and a 0.8 M NaCl buffer solution, the buffer solution was removed, and the resin was maintained for use. Then, 4 mL of a crude enzyme solution was added (the enzyme solution was prepared by dissolving a crude enzyme powder in 0.2 M PB 8.0 and a 0.8 M NaCl buffer solution to achieve a protein content of 25 mg/mL, with a corresponding cofactor PLP), cultured with the epoxy resin on a rail shaker at 25°C for 20 h, taken out, and subjected to standing and incubation at 4°C for 48 h. Washing was performed with 20 mM PB 8.0 for 3 times to obtain an immobilized transaminase containing 61 mg/g of a protein.

A test was carried out by a test means same as that in Example 1. Test results are shown in Table 11.

**Table 11**

| | | | | | |
|---|---|---|---|---|---|
| | Example 10 | Example 11 | Example 12 | Example 13 | Example 14 |
| Enzyme loading mount (mg/g) | 44 | 52 | 55 | 48 | 63 |
| Cycle number | 10 | 10 | 10 | 11 | 11 |
| Conversion rate (%) | >90% | >95% | >95% | >90% | >95% |

From the above description, it can be seen that the embodiments of the present application achieve the following technical effects.

The LX-109S epoxy resin is used as the epoxy resin carrier in the present application, such that on the basis of characteristics of the carrier itself, an immobilization effect of the carrier on the enzyme is more stable, and covalent bonding with the enzyme is firm without affecting the activity of the enzyme itself.

The statements above are merely preferred embodiments of the present application and are not intended to limit the present application, and for persons skilled in the art, various modifications and alterations of the present application can be made. Any modifications, equivalent substitutions, improvements and the like that are made within the spirit and principles of the present application shall be included in the scope of protection of the present application.

## Claims

1. An immobilized enzyme, wherein the immobilized enzyme comprises an epoxy resin carrier and an enzyme, the enzyme and the epoxy resin carrier are linked by a covalent bond, and the epoxy resin carrier is an LX-109S epoxy resin.

2. The immobilized enzyme according to claim 1, wherein the enzyme is selected from any one of a transaminase, a D-lactate dehydrogenase, a cyclohexanone monooxygenase, a ketoreductase, an ene reductase, a nitrilase, an ammonia lyase, an amino acid dehydrogenase, an imine reductase, a lipase and their mutants, preferably the ammonia lyase is selected from any one of a phenylalanine ammonia lyase, preferably the amino acid dehydrogenase is selected from any one of a leucine dehydrogenase and a phenylalanine dehydrogenase.

3. The immobilized enzyme according to claim 2, wherein the ammonia lyase is selected from any one of a phenylalanine ammonia lyase.

4. The immobilized enzyme according to claim 2, wherein the amino acid dehydrogenase is selected from any one of a leucine dehydrogenase or a phenylalanine dehydrogenase.

5. The immobilized enzyme according to claim 1, wherein a loading amount of the enzyme is 30 mg/g ~ 70 mg/g.

6. The immobilized enzyme according to claim 1, wherein the immobilized enzyme further comprises a cofactor.

7. A preparation method for the immobilized enzyme according to any one of claims 1 to 6, wherein the preparation method comprises:
Step S1, mixing a first phosphate buffer solution with an enzyme to form an enzyme solution, the enzyme is a crude enzyme; and
Step S2, mixing the enzyme solution with an epoxy resin for an immobilization reaction and then washing with a second phosphate buffer solution, to obtain the immobilized enzyme, wherein the epoxy resin is an LX-109S epoxy resin.

8. The preparation method according to claim 7, wherein a volume ratio of the enzyme solution to the epoxy resin is 3:1 to 5:1.

9. The preparation method according to claim 7, wherein a **pH** value of the first phosphate buffer solution is 7.0 to 8.0, the first phosphate buffer solution comprises sodium chloride; preferably, a concentration of the sodium chloride in the first phosphate buffer solution is 1±0.2 mol/L; and
a pH value of the second phosphate buffer solution is 7.0 to 8.0, the second phosphate buffer solution does not comprises sodium chloride.

10. The preparation method according to claim 7, wherein the Step S2 comprises:
mixing the enzyme solution and the epoxy resin at 10°C to 20°C, and culturing on a shaker for 16 hours to 24 hours, and then standing for 40 hours to 48 hours, to obtain an immobilized system; and
using the second phosphate buffer solution to wash the immobilized system, to obtain the immobilized enzyme.

11. An application of the immobilized enzyme according to any one of claims 1 to 6, wherein the application comprises applying the immobilized enzyme as a catalyst in a continuous catalytic reaction.
